Europäisches Patentamt

European Patent Office

Office européen des brevets

(11) Publication number: **0 442 558 A1**

# EUROPEAN PATENT APPLICATION

(21) Application number: **91200190.6**

(22) Date of filing: **31.01.91**

(51) Int. Cl.⁵: **C12N 9/20, C12P 7/64,**
C11C 3/08, C11C 3/10,
A23D 9/00

The applicant has filed a statement in accordance with Rule 28 (4) EPC (issue of a sample only to an expert). Accession number of the deposit: CMI 335426 (CMICC).

The applicant has subsequently filed a sequence listing and declared, that it includes no new matter.

(30) Priority: **12.02.90 EP 90301457**

(43) Date of publication of application:
**21.08.91 Bulletin 91/34**

(84) Designated Contracting States:
**BE CH DE DK ES FR GB IT LI NL SE**

(71) Applicant: **UNILEVER NV**
**Burgemeester s'Jacobplein 1 P.O. Box 760**
**NL-3000 DK Rotterdam(NL)**
(84) **BE CH DE DK ES FR IT LI NL SE**

Applicant: **UNILEVER PLC**
**Unilever House Blackfriars P.O. Box 68**
**London EC4P 4BQ(GB)**
(84) **GB**

(72) Inventor: **Charton, Emmanuelle**
**Unilever Research Laboratory, Colworth House**
**Sharnbrook, Bedford MK44 1LQ(GB)**
Inventor: **Slabas, Antoni Ryszard**
**Unilever Research Laboratory, Colworth House**
**Sharnbrook, Bedford MK44 1LQ(GB)**
Inventor: **Macrae, Alasdair Robin**
**Unilever Research Laboratory, Colworth House**
**Sharnbrook, Bedford MK44 1LQ(GB)**
Inventor: **Sidebottom, Christopher Michael**
**Unilever Research Laboratory, Colworth House**
**Sharnbrook, Bedford MK44 1LQ(GB)**

(74) Representative: **van der Toorren, Johannes, Drs. et al**
**UNILEVER N.V. Patent Division Postbus 137**
**NL-3130 AC Vlaardingen(NL)**

(54) **Fatty acid-specific lipase.**

(57) The production, isolation and partial characterisation of a new lipase from *Geotrichum candidum* is described, as well as several uses for it. It is called lipase B and is extremely specific for 9-cis fatty acids. The *Geotrichum candidum* used was deposited under the Budapest Treaty at the Culture Collection of the CAB International Mycological Institute (CMI CC) on 17 October 1989 under No. CMI 335426. Use is made from the option given under Rule 28(4) EPC. IPC⁵: C12N-9/20

# FATTY ACID-SPECIFIC LIPASE

Lipases are well-known in the literature. As many enzymes lipases can differ in their specificity, for example, the specificity for the $\alpha$- or $\beta$-ester group of triglycerides or the specificity for differences in the fatty acid chain in the triglycerides. Publications are known on lipases having a relatively high specificity for glycerol esters of 9-cis-unsaturated fatty acids such as oleic acid (9-cis-octadecenoic acid) and linoleic acid (9-cis-12-cis-octadecadienoic acid), see for example C. Franzke c.s. in Die Nahrung 17 (1973, No. 2) 171-184, S. Okumura c.s. in Agr. Biol. Chem. 40 (1976, No. 4) 655-660, M.W. Baillargeon c.s. in Appl. Microbiol. Biotechnol. 30 (1989) 92-96, and the review given by R.G. Jensen in Lipids 9 (1974, No. 3) 149-157. The maximum specificity described in these publications is 99% oleic acid (O) and 1% palmitic acid (P) from the oleate-rich glyceryl 2-palmitate-1,3-dioleate (OPO) (see Alford in J. Lipid Res. 5 (1964) 390-394, Table 4, but see below in this specification the discussion of the results given in Table IV), 94.0% O+L (L= linoleic acid) and 3.1% P produced from sunflower oil, 94.4% O+L and 4.6% P produced from arachidic oil (see Franzke c.s. in Zbl. Pharm 111 (1972) 1025-1033, Table 3), 93-93.3% O+L and 5.7-5.9% P produced from olive oil (see Kroll c.s. in Pharmazie 28 (1973) 263-269 in Table 5), and for *Geotrichum candidum* Link (ATCC 34614) a ratio of O/P = 100:25 and for *G. candidum* a O/P ratio of 100:4 based on the kinetics of competitive lipolysis of fatty acid methyl esters (see Aneja R. in JAOCS 64 (1987) 645). The only esters of fatty acids with a 9-trans double bond that are being hydrolysed at a reasonable rate are esters of 9-trans-12-cis-octadecadienoic acid (see R.G. Jensen & R.E. Pitas in Lipids Vol. 1, edited by R.Paoletti c.s., Raven Press, New York (1976) 141-146.

It was also described in e.g. the Franzke 1973 reference and the Jensen review that such specific 9-cis fatty acid lipases hydrolyze 11-cis fatty acids such as vaccenic acid and 6-cis fatty acids only at a much slower rate than 9-cis fatty acids. Examples of 6-cis fatty acids are petroselenic acid (6-cis-octadecenoic acid) and gamma-linolenic acid (6-cis-9-cis-12-cis-octadecatrienoic acid).

From the literature it is clear that the fatty acid specificity is found for both glycerides and other esters like methyl or butyl esters.

The present invention is based on the finding of a new *G. candidum* that produces a mixture of two lipases each of which having a different 9-cis fatty acid specificity. It was further found that one of these lipases was not selective for 9-cis fatty acids and the other one was extremely specific for 9-cis fatty acids. In this specification the specific lipase will be referred to as "lipase B" and the non-specific lipase as "lipase A".

Both lipase A and lipase B can be used in enzymatic transesterifications, using a lipase as catalyst, which reactions are well known. Examples of these reactions can be found in e.g. GB-B-1577933, in which in e.g. Example 11 a *G. candidum* lipase is described having a relatively high specificity for the exchange of 9-cis-containing fatty acid residues.

The present lipase B is a fraction of the extracellular lipases produced by a strain of *G. candidum*, which microorganism has been deposited at the Culture Collection of the CAB International Mycological Institute (CMI CC) on 17 October 1989 under No. CMI 335426. The isolation of this lipase B will be described below. In view of its very specific properties lipase B is an important new enzyme and both the compound itself and its use in various reactions, e.g. in the preparation of very pure oleic acid, are embodiments of the present invention. It should be noted that in J. Biochem. 106 (1989) 383-388; "cDNA Molecular Cloning of *G. candidum* Lipase" Shimada c.s. describe the cDNA sequence of a lipase gene. From the nucleotide sequence they deduced the mature *G. candidum* lipase to be a 544-amino acid polypeptide with a molecular weight of 59,085 (see page 387 and Figure 4 on page 386). However, on page 383 they state that the lipase used is the lipase from *G. candidum* ATCC 34614, which they described in reference 3 of that article (= Tsujisaka c.s. in Agr. Biol. Chem. 37 (1973, No. 6) 1457-1464) as the *G. candidum* Link lipase. This "ATCC 34614" lipase is the same as used by Aneja (see the (1987) reference above) having a hydrolysis ratio of O/P = 100:25, which means that they used a lipase which is much less specific than the lipase B according to the present invention.

Thus the present invention relates to this new lipase B having an extremely high specificity for 9-cis fatty acid esters, the latter meaning in this specification fatty acids containing an $-O-CO-(CH_2)_7-CH=CH-R$ group in which the olefinic group is a cis double bond and R may be a saturated or unsaturated hydrocarbon group, such as a methyl, ethyl, hexyl, octyl group or up to a C30 group and including unsaturated groups such as the remaining parts of linoleic and $\alpha$-linolenic (9-cis-12-cis-15-cis-octadecatrienoic acid). The R-group may also be branched or contain one or more cyclic radicals or hydroxy or epoxy groups, provided that the branching or substitution does not interfere with the lipase activity on the esterified $-O-CO-(CH_2)_7-CH=CH-$ part of the $-O-CO-(CH_2)_7-CH=CH-R$ group or on the esterification of the

HO-CO-$(CH_2)_7$-CH = CH-R fatty acid.

The invention provides an essentially pure lipase B, as described in this specification, having a specificity defined as a release of oleic acid and palmitic acid in a ratio of oleic acid : palmitic acid of more than 100 : 3 in a comparative test with methyl oleate and methyl palmitate, whereby about 200-400 units of lipase are used at 40° C during 15 minutes for the hydrolysis of 20 ml of an oil-in-water emulsion containing 1% of a mixture of methyl oleate and methyl palmitate in a ratio of between 40:60 and 60:40 and an aqueous phase having a pH of about 8.

An example of such lipase B has the following additional properties:
- in its glycosylated form it has a molecular weight of about 58,3 kDa determined by SDS-PAGE as described below in Example 2,
- it has an amino acid composition comprising about 5.9% of Ala, 3.7% of Arg, 15.9% of (Asn and Asp), 9.3% of (Gln and Glu), 5.3% of Gly, 3.4% of His, 3.6% of Ile, 9.2% of Leu, 4.0% of Lys, 2.4% of Met, 7.0% of Phe, 6.2% of Pro, 8.3% of Ser, 5.4% of Thr, 5.9% of Tyr, and 4.5% of Val, whereby the internationally accepted three-letter abbreviation of the amino acids is used, the percentages are by weight of the total amino acid composition of a hydrolysate of lipase B and the amounts of Cys and Trp are not determined, and
- it has an amino acid sequence containing the following partial amino acid sequences Gly-Ile-Pro-Phe-Ala-Asp-Pro-Pro and Gly-Leu-Glu-Trp-Val-Ser-Asp-Asn-Ile-Ala-Asn-Phe-Gly-Gly-Asp.

Preferably its amino acid sequence contains at least one partial amino acid sequence selected from the group consisting of Val-Pro-Tyr-Ile-Thr-Gly, Asn-Gln-Glu-Asp-Glu-Gly and Thr-Ile-Leu-Ala-Pro-Val.

In this specification percentages and amounts are by weight unless otherwise indicated and "essentially pure lipase B" means an active lipase containing at least 95%, preferably at least 98% and more preferably at least 99% of lipase B and thus not more than 5%, preferably not more than 2%, and more preferably not more than 1% of other active lipases, the percentages being calculated on the total amount of active lipase-(s) present. Thus other ingredients may be present in the crude lipase mixture. With "active lipase" is meant a lipase that is still capable of hydrolysing fatty acid esters. Thus lipase that is inactivated for one or other reason, e.g. by denaturation, is no longer counted as active lipase.

Uses of lipase B.

The invention further relates to the use of lipase B as defined above for the selective hydrolysis of 9-cis fatty acids present in mixed esters thereof, in particular for the production of very pure oleic acid by hydrolysis of esters containing fatty acid residues of oleic acid and other fatty acids, the latter in particular being fatty acids not containing a divalent 9-decenoyl (-O-CO-$(CH_2)_7$-CH = CH-) group, followed by separating the oleic acid, or other 9-cis fatty acid(s), from the hydrolysis mixture. Such hydrolysis for producing oleic acid from high oleic sunflower oil was described in WO-A-8903419. The present lipase B is more specific than the lipases described in that publication.

Thus lipase B can be used for treating mixtures of triglycerides having a high content of oleic acid residues such as olive oil and tallow for the preparation of oleic acid of very high purity, which finds application in e.g. the pharmaceutical field. In addition to glycerol esters of oleic acid, however, oleic acid esters of other oligo- or mono-alcohols can also be used as starting material for the production of oleic acid. Like the tri-, di- and mono-glycerides these esters of other alcohols may also contain fatty acids moieties other than oleic acid residues, or may be mixtures of esters of the same or different alcohols with several fatty acids, particularly if these other fatty acids are saturated or contain one or more olefinic groups between the ninth carbon atom and the carboxyl group or have a 9-trans olefinic group. Also ester mixtures of impure oleic acid, e.g. those containing esterified isomers of oleic acid as can be obtained by hydrogenating unsaturated fatty oils, can be used as oleic acid source. Then only the real oleic acid (9-cis-octadecenoic acid) is hydrolysed by lipase B contrary to the isomers.

Lipase B can also be used for producing linoleic acid or mixtures of linoleic acid and oleic acid from oils containing esters of these fatty acids in admixture with other esterified fatty acids by selective hydrolysis. Similarly pure 9-cis-palmitoleic acid can be produced from sources containing it. Thus only the 9-cis fatty acid esters will be hydrolysed, the other (saturated, trans- or site-isomer) residues are not or practically not hydrolysed.

Other uses of lipase B include the interesterification of mixtures of triglycerides with the aim of selectively exchanging 9-cis fatty acids or for the introduction of a 9-cis fatty acid into an ester by reacting such 9-cis fatty acid with such ester, the introduction of a 9-cis fatty acid into a partial ester by reacting such 9-cis fatty acid with such partial ester, or the production of an ester or partial ester from a 9-cis fatty acid and an alcohol containing one or more hydroxy groups by reacting such 9-cis fatty acid with such alcohol under conditions generally known for esterification.

For example, one might use the lipase B for replacing a mono-unsaturated 9-cis fatty acid residue, e.g. the oleic acid residue, in a triglyceride by a poly-unsaturated fatty acid residue with at least a 9-cis double bond (but not those also having a 6-cis double bond), e.g. residues of the essential fatty acids such as linoleic and $\alpha$-linolenic acid, or *vice versa*. A specific application of this use of lipase B is to catalyse exchange of 9-cis unsaturated fatty acid residues in symmetrical triglycerides such as POP, POS, SOS, PLP, PLS, and SLS, or LPL, LSL, LPO, LSO, OPO or OSO, with L, O and P as defined above and S meaning the alkylcarboxyl residue of a saturated fatty acid, e.g. palmitic acid or stearic acid. The replacement of mono-unsaturated by poly-unsaturated fatty acid residues will result in triglycerides with an increased content of poly-unsaturated fatty acid residues, which are desirable ingredients in combatting high cholesterol levels in human blood.

Provided that the fatty acids to be hydrolysed or esterified contain at least a divalent 9-decenoyl (-O-CO-$(CH_2)_7$-CH=CH-) group, the chain length of the mono- or poly-unsaturated fatty acid residues in the esters is not so important, although it is preferred to use C16-C18 fatty acid residues. Most preferred fatty acid residues comprise oleic and linoleic acid residues. The hydrolysis or (inter)esterification can be carried out in any way described for enzymatic transesterification (see for example GB-B-1577933, EP-B-0034065, EP-B-0069599 and EP-B-0093602). It is possible to carry out the reaction either batch-wise or in a continuous way. The presence of a very small amount of water in the reaction system is advantageous for maintaining the enzyme-activity. A water desiccant means can be added to the system in order to decrease the damaging effect of the presence of water in the system, as described in EP-B-0064855.

The reaction time is preferably not longer than 2 hours as has been pointed out in EP-B-0093602. The reaction temperature can be chosen between 0 and 80° C, preferably between 40 and 60° C.

The invention also relates to products obtainable by the use of lipase B, which products can be obtained by one of the uses of lipase B described above, as well as to edible products containing such products. Such edible products comprise food products, for example chocolate compositions and baby fats, as well as margarines and other spreads.

Although the invention is illustrated with lipase B as isolated from the *Geotrichum candidum* (CMI CC 335426), it will be clear that the invention also comprises modifications of this lipase B having the same or different number of amino acid residues and containing one or more differences in amino acid composition, provided that the resulting polypeptide is still active as lipase and has the same or better specificity as the above described lipase B. Also the degree of glycosylation is not important provided that the high specificity of the lipase is not essentially decreased.

The invention is exemplified with the following examples.

## Example 1. Production of *Geotrichum candidum* lipase in laboratory scale fermenters

Spores of *Geotrichum candidum* isolated from milk (CMI CC 335426, see above) were inoculated to a medium containing per litre of distilled water: 1.5 g KH2PO4, 1.0 g NH4Cl, 1.2 g MgSO4.7H2O, 20.0 g Difco Beta Lab Yeast Extract, 25.0 g olive oil, 17 mg ZnSO4, 17 mg MnSO4, 17 mg FeSO4. All media constituents were sterilized together at 121° C for 30 minutes. The starting parameters for running the fermentation were as detailed below:

```
Temperature                              30°C
Agitation using a series of
3 Rushton pitched blades                 400 r.p.m.
Air flow                                 0.16 v/v/m
Oxygen supplement                        0.32 v/v/m
pH                                       6.4
```

The pH of the fermentation was maintained at 4.5 during the period of lipase production using 0.88 ammonia solution. The level of biomass generated during the vegetative growth of *Geotrichum* necessitated the two parameters aeration and agitation to be altered, such that the high oxygen demands could be met and the dissolved oxygen maintained above 20%. These parameters were increased to a combined air and oxygen flow rate of 0.83 v/v/m and a maximum agitation rate of 800 r.p.m., depending on the degree of foaming generated. The foam was also controlled by the use of Silcolapse 5000TM (ex ICI); about 100 p.p.m. were added at the time of inoculation (TM means Trade Mark).

The largest increase in titre of lipase found within the supernatant appeared to coincide with sporulation of culture. The normal time for harvesting from the point of inoculation was anything from 24-32 hours. It was also found advantageous to supplement the media at approximately 24 hours with a further 0.25 g/l magnesium sulphate.

Example 2. Purification and separation of the two extracellular lipases from *Geotrichum candidum*

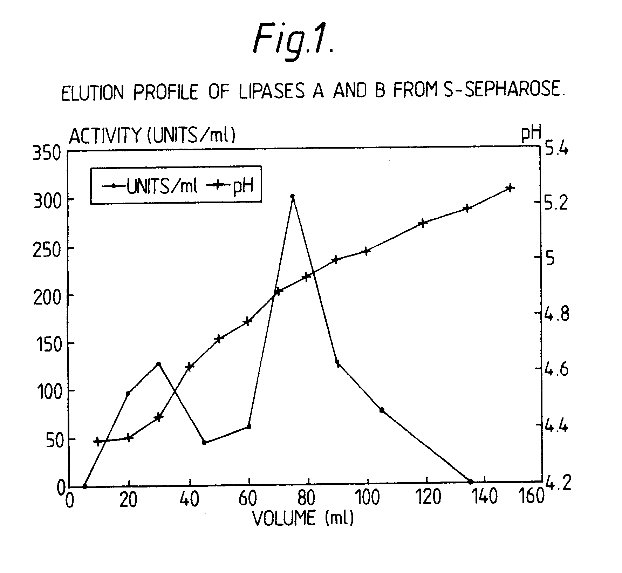

The fermentation culture was filtered using Whatman No.1TM paper giving 4 liter of crude enzyme solution containing two lipases, one being the specific lipase B. After pH adjustment to 5.8 and addition of 0.5% w/v Triton X-100TM this solution was bound to 200 ml of Q Sepharose Fast FlowTM (ex Pharmacia) equilibrated in 50 mM sodium phosphate buffer (pH to 5.8). The enzymes were eluted from the anion exchanger with a 1 liter salt gradient (0 to 0.5 M NaCl in 50 mM sodium phosphate buffer (pH to 5.8), flow rate 1 ml/min) in one peak of biological activity (final volume 300 ml). A sample of this concentrated lipase solution (30,000 units) was diluted 50-50 in water and bound on 5 ml of the cation exchanger S Sepharose Fast FlowTM (ex Pharmacia) equilibrated in 20 mM Piperazine.HCl pH 4.0. A 200 ml pH gradient from 4.0 to 5.3 in 20 mM Piperazine.HCl generated by FPLC at a flow rate of 2 ml/min resulted in two peaks of activity (Fig.1). The first peak, eluted between pH 4.2 and 4.5, corresponded to "lipase A" and was collected in the first 35 ml of eluate, giving 1500 units of enzyme. The second peak, eluted between pH 4.75 and 5.1, corresponded to the pure specific "lipase B" (13,000 units collected from 50 to 115 ml of eluate). The elution profile of lipases A and B from S-SepharoseTM is given in Fig. 1.

The denatured molecular weight of lipase B determined by SDS-PAGE with appropriate standards was 58.3 kDa. After Endo H treatment for deglycosylation the molecular weight of lipase B found by this method was decreased to 54.0 kDa.

The native molecular weight of lipase B determined by gel filtration using two calibrated, in-line Superose 12TM FPLC columns (ex Pharmacia) was 48.9 kDa. The columns were equilibrated in 50 mM sodium phosphate buffer (pH to 7.5, 0.15 M NaCl and initialy coated with bovine serum albumin to prevent non-specific binding. The following standards (ex Pharmacia) were used to calibrate the column: catalase (232 kDA), aldolase (158 kDA), phosphorylase (97.4 kDa), ovalbumin (45 kDa), - chymotrypsinogen (25 kDa) and ribonuclease (13.7 kDa). Samples were loaded via a 200 l loop, eluted at 0.3 ml.min$^{-1}$ and fractions of 200 l were collected. Lipase was located by absorbance at 280 nm and enzyme activity.

A pI of 4.50 (both before and after treatment with Endo H used for deglycosylation) was determined by isoelectric focussing. This was performed in a PHASTTM gel system (ex Pharmacia) using calibrated pH 4.0-6.5 gels according to manufacturer's instructions.

Example 3. The specificity of lipase B in comparison with other *Geotrichum candidum* lipases.

First the activities of the following lipases from *G. candidum* were investigated using a standard olive oil assay.

I : AMANO Pharmaceutical Co. Ltd, Nagoya, JAPAN
II : BIOCATALYSTS Ltd, Pontypridd, UK
III : ATCC 34614, Rockville, Maryland, USA
IV : NRRLY552 Northern Utilization Research and Development Division, US Department of Agriculture, Peoria, Illinois, USA
V : NRRLY553 (same source)
VI : COLWORTH LIPASE, CRUDE
VII : COLWORTH LIPASE A
VIII : COLWORTH LIPASE B

Each sample of enzyme solution was added to 20 ml of an oil-in-water emulsion containing 5% olive oil, 2% gum arabic and 0.5% CaCl$_2$. The emulsion was made by sonication for 3 minutes at speed 6 on an Ultrasonic sonifier marketed by Lucas Dawe. The standard olive oil assay was carried out at 40°C and the release of fatty acids was measured by automatic titration (RadiometerTM Copenhagen) with 0.1 NaOH to pH 8. A curve of amount of titrant against time was obtained. The activity of the lipase was calculated from the maximum slope of this curve. One unit of enzyme is defined as the amount of enzyme that releases 1 mol of fatty acid from olive oil in one minute under the conditions specified above.

Then different substrates were chosen for further study of specificity, whereby the 5% olive oil in the emulsion was replaced by the substrate and in the amount given below.

- 1% palm oleine

The fatty acid composition of the palm oleine used in these experiments is shown in Table I. The reactions were catalyzed with 30 units of lipase solutions at 40°C for 15 minutes. Digestion was terminated with the addition of 1 ml of 1 M HCl to a 2 ml sample. The digestion products were extracted with petroleum ether (b.p. 40-60°C) and separated by TLC on 20x20 cm glass plates coated with 1 mm silica gel G obtained from Anachem (ex Luton, GB). Development was in petroleum ether (b.p. 40-60°C) / diethyl ether formic acid in a ratio of 70:30:1. Bands were visualized under UV after spraying with 0.01% phloxinTM in 50% methanol. The fatty acids bands were extracted with diethyl ether and methylated using 14% boron trifluoride in methanol as a catalyst. The composition of the released fatty acids was calculated as the difference between the 15 minutes value and the initial amount of free fatty acids in the emulsion. The results are given in Table II and show the high specificity of lipase B for unsaturated substrates: the fatty acids released from palm oleine by lipase B contained only 0.4% palmitic acid. In contrast, with the other *Geotrichum candidum* lipases, substantial amounts of palmitic acid were released from palm oleine.

- 0.5% methyl esters of oleic, palmitic, elaidic and vaccenic acids; vaccenic acid = 11-cis-octadecenoic acid

The reactions were carried out for 3 minutes at 30°C, using 5 units of enzyme solutions. The rate of hydrolysis of each methyl ester was calculated relatively to that of methyl oleate and was expressed as a percentage. The results are given in Table III. In order to confirm the very low activity of lipase B on palmitic acid methyl ester, the reaction was catalyzed with 200 units of enzyme, and still gave a rate close to 0.

- 1% equimolar mixture of Me-oleate and Me-palmitate

The hydrolysis was catalyzed by 309 olive oil units of lipase B. The fatty acid composition of a sample was determined after 15 minutes reaction as described above for palm oleine, the only difference being the separation of the free fatty acids from the remaining methyl esters by thin layer chromatography (petroleum ether (b.p. 40-60°C) / diethyl ether / formic acid 90:10:1). The FFA composition was: 1% palmitate, 99% oleate.

The specificity of lipase B was also investigated on emulsions of the following substrates:
- 1% PPO = glyceryl 2,3(1)-dipalmitate-1(3)-oleate,
- 1% POP = glyceryl 1,3-dipalmitate-2-oleate,
- 1% SOS = glyceryl 1.3-distearate-2-oleate

at 40°C in 2% gum arabic containing 0.5% CaCl2. The reaction was carried out for 15 minutes, using 30 olive oil units of lipase B. The analysis of the free fatty acids released from the triglycerides was done as described above for palm oleine. The results are given in Table IV and confirm the high specificity of lipase B for unsaturated substrates. In particular, the results expressed as the ratio oleate : palmitate/stearate with lipase B and PPO, POP, and SOS are much better than those described in Table 4 of Alford c.s. (1964) mentioned on page above:

|  | **lipase B** | **Alford lipase** |
|---|---|---|
| **POP** | 98.9 : 1.1 | 80 : 20 |
| **PPO** | 99.5 : 0.5 | 65 : 35 |
| **SOS** | 99.9 : 0.1 | 98 : 2 |

Table I: Fatty acid composition of palm oleine described in the text

| FATTY ACIDS GROUP | 12:0 | 14:0 | 16:0 | 16:1 | 18:0 | 18:1 | 18:2 | 18:3 and 20:0 |
|---|---|---|---|---|---|---|---|---|
| AMOUNT IN PALMOLEINE (%) | 0.4 | 1.0 | 40.2 | 0.2 | 4.3 | 42.6 | 10.4 | 0.9 |

- - - - - - - - -

Table II: Composition (%) of the fatty acids released from palm oleine by various *Geotrichum candidum* lipases under the conditions described in the text

| LIPASES | I | II | III | IV | V | VI | VII | VIII |
|---|---|---|---|---|---|---|---|---|
| **FATTY ACIDS:** | | | | | | | | |
| 16:0 | 21.0 | 34.7 | 41 | 7.8 | 18.9 | 20.5 | 40.5 | 0.4 |
| 18:0 | 0.7 | 0.5 | 1.0 | 0.0 | 0.0 | 0.2 | 0.9 | 0.0 |
| 18:1 | 67.6 | 52.8 | 44.8 | 77.3 | 69.5 | 66.6 | 46.2 | 83.9 |
| 18:2 | 10.7 | 12.0 | 13.2 | 14.9 | 11.6 | 12.7 | 12.4 | 15.7 |

- - - - - - - - -

Table III: Relative rates of hydrolysis of fatty acids methyl esters by various *Geotrichum candidum* lipases under the conditions described in the text

| LIPASES | I | II | III | IV | V | VI | VII | VIII |
|---|---|---|---|---|---|---|---|---|
| **ESTERS OF:** | | | | | | | | |
| OLEATE | 100 | 100 | 100 | 100 | 100 | 100 | 100 | 100 |
| ELAIDATE | 20.2 | 30.2 | 15.2 | 41.1 | 34.8 | 15.9 | 23.9 | 0.7 |
| VACCENATE | 10.6 | 15.9 | 21.0 | 22.2 | 26.1 | 8.5 | 13.1 | $\leq$0.01 |
| PALMITATE | 45.7 | 66.7 | 42.9 | 54.4 | 34.4 | 26.8 | 64.2 | $\leq$0.001 |

- - - - - - - - -

Table IV: Composition (%) of the fatty acids released from pure triglycerides by Lipase B under the conditions described in text

| FATTY ACIDS | 16:0 | 18:0 | 18:1 |
|---|---|---|---|
| **TRIGLYCERIDES:** | | | |
| POP | 1.1 | | 98.9 |
| PPO | 0.5 | | 99.5 |
| SOS | | 0.1 | 99.9 |

- - - - - - - - -

```
SEQ ID NO:                              1
SEQUENCE TYPE:                          partial amino acid sequence
SEQUENCE LENGTH:                        8

STRANDEDNESS:                           not applicable
TOPOLOGY:                               linear
MOLECULE TYPE:                          oligopeptide

ORIGINAL SOURCE
ORGANISM:                               mould
IMMEDIATE EXPERIMENTAL SOURCE:          Geotrichum candidum
NAME OF CELL LINE:                      Geotrichum candidum CMI 335426

FEATURES:

PROPERTIES:                             the oligopeptide forms part of
                                        lipase B isolated from the
                                        organism as deposited, which
                                        lipase B has a very high
                                        specifity for the hydrolysis
                                        of 9-cis fatty acids

Gly-Ile-Pro-Phe-Ala-Asp-Pro-Pro
1               5           8
```

*-*-*-*-*

```
SEQ ID NO:                              2
SEQUENCE TYPE:                          partial amino acid sequence
SEQUENCE LENGTH:                        15

STRANDEDNESS:                           not applicable
TOPOLOGY:                               linear
MOLECULE TYPE:                          oligopeptide

ORIGINAL SOURCE
ORGANISM:                               mould
IMMEDIATE EXPERIMENTAL SOURCE:          Geotrichum candidum
NAME OF CELL LINE:                      Geotrichum candidum CMI 335426

FEATURES:

PROPERTIES:                             the oligopeptide forms part of
                                        lipase B isolated from the
                                        organism as deposited, which
                                        lipase B has a very high
                                        specifity for the hydrolysis
                                        of 9-cis fatty acids

Gly-Leu-Glu-Trp-Val-Ser-Asp-Asn-Ile-Ala-Asn-Phe-Gly-Gly-Asp
1               5                   10                  15
```

*-*-*-*-*

| | |
|---|---|
| SEQ ID NO: | 3 |
| SEQUENCE TYPE: | partial amino acid sequence |
| SEQUENCE LENGTH: | 6 |
| STRANDEDNESS: | not applicable |
| TOPOLOGY: | linear |
| MOLECULE TYPE: | oligopeptide |
| ORIGINAL SOURCE | |
| ORGANISM: | mould |
| IMMEDIATE EXPERIMENTAL SOURCE: | *Geotrichum candidum* |
| NAME OF CELL LINE: | *Geotrichum candidum* CMI 335426 |
| FEATURES: | |
| PROPERTIES: | the oligopeptide forms part of lipase B isolated from the organism as deposited, which lipase B has a very high specifity for the hydrolysis of 9-cis fatty acids |

Val-Pro-Tyr-Ile-Thr-Gly
1                            6

*-*-*-*-*

| | |
|---|---|
| SEQ ID NO: | 4 |
| SEQUENCE TYPE: | partial amino acid sequence |
| SEQUENCE LENGTH: | 6 |
| STRANDEDNESS: | not applicable |
| TOPOLOGY: | linear |
| MOLECULE TYPE: | oligopeptide |
| ORIGINAL SOURCE | |
| ORGANISM: | mould |
| IMMEDIATE EXPERIMENTAL SOURCE: | *Geotrichum candidum* |
| NAME OF CELL LINE: | *Geotrichum candidum* CMI 335426 |
| FEATURES: | |
| PROPERTIES: | the oligopeptide forms part of lipase B isolated from the organism as deposited, which lipase B has a very high specifity for the hydrolysis of 9-cis fatty acids |

Asn-Gln-Glu-Asp-Glu-Gly
1                            6

*-*-*-*-*

```
SEQ ID NO:                            5
SEQUENCE TYPE:                        partial amino acid sequence
SEQUENCE LENGTH:                      6

STRANDEDNESS:                         not applicable
TOPOLOGY:                             linear
MOLECULE TYPE:                        oligopeptide

ORIGINAL SOURCE
ORGANISM:                             mould
IMMEDIATE EXPERIMENTAL SOURCE:        Geotrichum candidum
NAME OF CELL LINE:                    Geotrichum candidum CMI 335426

FEATURES:

PROPERTIES:                           the oligopeptide forms part of
                                      lipase B isolated from the
                                      organism as deposited, which
                                      lipase B has a very high
                                      specifity for the hydrolysis
                                      of 9-cis fatty acids

Thr-Ile-Leu-Ala-Pro-Val
1                       6
```

$$*-*-*-*-*$$

## Claims

1. Essentially pure lipase B, as described in this specification, having a specificity defined as a release of oleic acid and palmitic acid in a ratio of oleic acid : palmitic acid of more than 100 : 3 in a comparative test with methyl oleate and methyl palmitate, whereby about 200-400 units of lipase are used at $40°C$ during 15 minutes for the hydrolysis of 20 ml of an oil-in-water emulsion containing 1% of a mixture of methyl oleate and methyl palmitate in a ratio of between 40:60 and 60:40 and an aqueous phase having a pH of about 8.

2. Lipase B according to claim 1 having the following additional properties:
   - in its glycosylated form it has a molecular weight of about 58,3 kDa determined by SDS-PAGE as described above in the specification,
   - it has an amino acid composition comprising about 5.9% of Ala, 3.7% of Arg, 15.9% of (Asn and Asp), 9.3% of (Gln and Glu), 5.3% of Gly, 3.4% of His, 3.6% of Ile, 9.2% of Leu, 4.0% of Lys, 2.4% of Met, 7.0% of Phe, 6.2% of Pro, 8.3% of Ser, 5.4% of Thr, 5.9% of Tyr, and 4.5% of Val, whereby the internationally accepted three-letter abbreviation of the amino acids is used, the percentages are by weight of the total amino acid composition of a hydrolysate of lipase B and the amounts of Cys and Trp are not determined, and
   - it has an amino acid sequence containing the following partial amino acid sequences Gly-Ile-Pro-Phe-Ala-Asp-Pro-Pro and Gly-Leu-Glu-Trp-Val-Ser-Asp-Asn-Ile-Ala-Asn-Phe-Gly-Gly-Asp.

3. Lipase B according to claim 1 or 2, having an amino acid sequence containing at least one partial amino acid sequence selected from the group consisting of Val-Pro-Tyr-Ile-Thr-Gly, Asn-Gln-Glu-Asp-Glu-Gly and Thr-Ile-Leu-Ala-Pro-Val.

4. Lipase B according to any one of claims 1-3, in which the content of active lipase B is at least 98%, preferably at least 99%, of the total amount of active lipase(s) present.

5. Process for the selective hydrolysis of 9-cis fatty acids present in mixed esters thereof in the presence of a lipase as a catalyst for the hydrolysis, which process comprises the use of lipase B claimed in any of claims 1-4.

6. Process according to claim 5, in which process very pure oleic acid (9-cis-octadecenoic acid) is produced by hydrolysis of esters containing fatty acid residues of oleic acid and other fatty acids, the latter in particular being fatty acids not containing a divalent 9-decenoyl $(-O-CO-(CH_2)_7-CH=CH-)$ group, followed by separating the oleic acid from the hydrolysis mixture.

7. A process for the interesterification of mixtures of triglycerides in the presence of a lipase as a catalyst for the interesterification, which process comprises the use of lipase B claimed in any of claims 1-4 with the aim of selectively exchanging 9-cis fatty acids.

8. A process for the introduction of a 9-cis fatty acid into an ester by reacting such 9-cis fatty acid with such ester in the presence of a lipase as a catalyst, which process comprises the use of lipase B claimed in any of claims 1-4.

9. A process according to claim 8, in which a 9-cis fatty acid is introduced into a partial ester by reacting such 9-cis fatty acid with such partial ester.

10. A process for the production of an ester or partial ester from a 9-cis fatty acid and an alcohol containing one or more hydroxy groups by reacting such 9-cis fatty acid with such alcohol in the presence of a lipase under conditions generally known for enzymatic esterification, which process comprises the use of lipase B claimed in any of claims 1-4.

11. Product obtainable by the use of lipase B claimed in any one of claims 1-4 as a catalyst for a hydrolysis, esterification or interesterification reaction.

12. Product according to claim 11 and obtained by a process as claimed in any of claims 5-10, optionally followed by a separation step.

13. Edible product containing a product according to claim 11 or claim 12.

# Fig.1.

ELUTION PROFILE OF LIPASES A AND B FROM S-SEPHAROSE.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | J. BIOCHEM., vol. 106, 1989, pages 383-388; Y. SHIMADA et al.: "cDNA molecular cloning of Geotrichum candidum lipase" * Whole document * | 1-11 | C 12 N    9/20<br>C 12 P    7/64<br>C 11 C    3/08<br>C 11 C    3/10<br>A 23 D    9/00 |
| Y | FR-A-2 340 979 (UNILEVER) * Claims; pages 2,3,4-7 * | 1,4-12 | |
| Y | EP-A-0 093 602 (UNILEVER) * Claims; pages 2,3 * | 1,4-12 | |
| Y | CHEMICAL ABSTRACTS, vol. 78, 5th February 1973, page 183, abstract no. 25750t, Columbus, Ohio, US; R.G. JENSEN et al.: "Specificity of Geotrichum candidum lipase with respect to double bond position in triglycerides containing cisoctadecenoic acids", & LIPIDS 1972, 7(11), 738-41 | 1,4-12 | |
| Y | CHEMICAL ABSTRACT, vol. 68, 1968, pages 7256-7257, abstract no. 75350b, Columbus, Ohio, US; T.A. MARKS et al.: "Studies on the specifiicity of a lipase system from Geotrichumm candidum", & LIPIDS 3(2), 143-6(1968) | 1.4-12 | TECHNICAL FIELDS SEARCHED (Int. Cl.5)<br><br>C 12 P<br>C 11 C<br>C 12 N |

The present search report has been drawn up for all claims

| Place of search | Date of completion of the search | Examiner |
|---|---|---|
| THE HAGUE | 16-05-1991 | DELANGHE L.L.M. |